Europäisches Patentamt

European Patent Office

Office européen des brevets

(19)

(11) Publication number: **0 1 1 0 407**
**A2**

(12) # EUROPEAN PATENT APPLICATION

(21) Application number: **83112034.0**

(22) Date of filing: **30.11.83**

(51) Int. Cl.³: **A 61 K 35/16, A 61 L 2/04**

(30) Priority: **02.12.82 US 446138**

(43) Date of publication of application: **13.06.84**
**Bulletin 84/24**

(84) Designated Contracting States: **AT BE CH DE FR GB IT LI LU NL SE**

(71) Applicant: **USV PHARMACEUTICAL CORPORATION, 1 Scarsdale Road, Tuckahoe New York (US)**

(72) Inventor: **Landaburu, Ricardo H., 1 Magnolia Drive, Rye Town New York (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath, Maximilianstrasse 58, D-8000 München 22 (DE)**

(54) **Hepatitis B and non-A-non-B-safe biological products.**

(57) Biological material is rendered non-infectious as to hepatitis B and non-A-non-B viruses by incubating the material with hepatitis B antibody, drying the incubated material, and then heating the dried material under conditions effective to render the HnAnBV non-infectious without adversely affecting the biological activity of the biological material.

EP 0 110 407 A2

## HEPATITIS B AND NON-A-NON-B-SAFE BIOLOGICAL PRODUCTS

The present invention relates to rendering biological products, and particularly plasma fractions, free of transmissible infectious hepatitis viruses, specifically hepatitis B virus ("HBV") and hepatitis non-A-non-B virus(es) ("HnAnBV").

Continuing concern for the safety of recipients of biological protein products has inevitably focused on the potential transmission of serum hepatitus during blood component therapy, which may be caused by the presence of even undetectable levels of infective viruses in the donor plasma pool. Though tested in each case by state of the art methods, it is widely understood that low but infectious levels of HBV may still occur undetectable in plasma, and no in vitro test has been devised which predicts the presence or absence of the HnAnBV.

The safety of some blood component products can be promoted by heating these proteins as stabilized solutions at 60°C for 10 hours. This method cannot be directly applied to i.e. those preparations used in the treatment of hemophilia (Factor VIII, Factor IX) since it results in substantial lessening or destruction of the product potency. In addition, it is not clear that such treatment is fully effective against both HBV and HnAnBV. Thus, there is still a need for a process for destroying HBV and HnAnBV activity in protein products, particularly plasma fractions such as Factor VIII, Factor IX, and all other products which lose biological activity when heated in aqueous solution.

U.S. Patent No. 4,346,073 describes the reduction of hepatitis B infectivity in biological products by incubating the products in vitro with an effective amount of hepatitis B immune globulin.  The hepatitis B immune globulin is derived from the plasma of persons exhibiting high titers of antibody to the hepatitis B surface antigen. The disclosed process, while highly useful, inherently co-administers in the immune globulin other antibodies in amounts which can (1) give rise to allotypic antibody response; (2) suppress endogenous recipient antibody formation; and (3) give rise to circulating immune complexes which can lead to further diseased states.  In addition, the patent does not address treatments of HnAnBV.

Abstracts by A. Rubinstein, Thrombosis and Haemostasis (July 12, 1981), pages 338 and 339, indicate that lyophilized Factor VIII and Factor IX can each be heated at 60°C for 10 hours without showing significant change in activity four hours after heating.  The author then speculates that such heating, perhaps for a longer duration, might be effective to inactivate "the hepatitis virus", though without even speculating as to which virus might be affected.  However, it can readily be demonstrated that such a heating regimen has essentially no effect on the activity of the hepatitis B virus; thus, these abstracts serve perhaps to acknowledge the problem but do not suggest its solution.

Briefly stated, the present invention comprises a process for preventing the tramsission of hepatitis B virus and hepatitis non-A-non-B virus(es) by an injectable biological product  containing said viruses, comprising

(a)   incubating the biological product in vitro in solution with an amount of hepatitis B antibody effective to render the biological product non-infectious with respect to hepatitis B,

0110407

(b)    drying the mixture formed in step (a) under conditions which are non-destructive to the biological product, and

(c)    heating the dried mixture formed in step (b) under conditions effective to render the biological product non-infectious with respect to hepatitis non-A-non-B, while retaining at least about 10% of the biological activity of the biological products.

The inventive process is also readily capable of providing biological products in which substantially more than 10% of the activity is retained, and in which the biological product suffers essentially no denaturation.

The present invention is broadly applicable to biological products containing hepatitis B virus ("HBV") and the virus(es) which are collectively known to those skilled in this art as hepatitis non-A-non-B virus(es) ("HnAnBV"), even where the HBV and HnAnBV are present below detectable levels.  The invention is also usable against other heat-susceptaible viruses, such as cytomegalovirus, Epstein-Barr virus, and other such viruses which will be apparent to those skilled in this art.  The biological products themselves include products derived from pooled plasma as well as from tissues.

The present invention is applicable to all types of plasma protein products for therapeutic as well as non-therapeutic uses.  Examples of such products are:

- Blood and blood fractions such as Antithrombin III, antihemophilic factor A (AHF, Factor VIII); prothrombin complex (Factors II, VII, IX and X); gamma globulin; albumin; and the like;

- Clinical diagnostic control sera containing human or animal (e.g., porcine or bovine) plasma protein components (e.g., albumin); and

- Placenta plasma proteins.

Of particular interest are products such as Antithrombin III, Factor VIII and Factor IX, however derived, and all other products which lose biological activity when heated in aqueous solutions thereof at conventional pasteurization conditions, e.g. 60°C for 10 hours.

In practice, the virus-bearing biological product is first treated in aqueous solution by adding an effective amount of hepatitis B antibody. The present invention will be described herein with particular reference to commercial-grade Factor VIII, which as one skilled in this art will recognize comprises an aqueous solution which has a certain small statistical probability of possessing hepatitis infectivity. Generally, the amount of antibody which is added to the solution will be in the range of about 0.1 to about 20 International Units ("IU") of hepatitis B antibody per milliliter of solution being treated. Depending on the degree of the HBV infectiveness, the amount of antibody can be in the range of 0.1 to 1.0 IU/ml. Antibody amounts of the order of 0.27 IU/ml have been found to be effective against HBV doses of $10^{3.5}$ $CID_{50}$ (Chimpanzee Infective Doses$_{50}$), the amount which will infect 50% of a sample population of chimpanzees) in commercial Factor VIII, while higher amounts of antibody, preferably 0.5 IU/ml, would be effective in an even higher percentage of cases. As used herein, "International Units" of HBV are as defined in the publication of F. Blaine Hollinger, E. Adam, D. Heiberg, J. L. Melnick: "Response to Hepatitis B Vaccine in a Yough Adult Population", Viral Hepatitis International Symposium, ed. W. Szmuness, H. J. Alter, J. E. Maynard, pp 451-466, Franklin Institute Press, Philadelphia, 1982.

Generally, the hepatitis B antibody can be derived by Cohn fractionation from the plasma of patients exhibiting high immune titer to the hepatitis B surface antigen. The hepatitis B antibody can be added as Hepatitis B Immune Globulin. As taught in U.S. Patent No. 4,346,073 to Aronson et al., Hepatitis B Immune Globulin is a preparation derived from the plasma of persons with high titers of antibody to hepatitis B surface antigen, and has been licensed by the Food and Drug Administration for administration to persons following exposure to small doses of hepatitis B virus. However, the amount of this Hepatitis B Immune Globulin required to provide a satisfactorily high amount of protective hepatitis B antibody can be on the order of 90 to 100 mg or higher. Amounts this high, administered repeatedly to the patient, expose the patient to the side effects mentioned above due to other antibodies in the immunoglobulin.

Thus, in a preferred embodiment of this invention the hepatitis B antibody is added as a monospecific antibody obtained by further purification of Hepatitis B Immune Globulin which contains one or more non-hepatitic antibodies. Such purification can be carried out by ion-exchange chromatography or other chemical absorption of the antibody followed by desorption, such as affinity chromatography employing the HBV or, preferably, derived antigens thereof, on a solid substrate. This separatory process can be applied to plasma, to Cohn Fraction II, to antibody-containing side fractions of Cohn fractionation, or to other partially purified compositions including the Hepatitis B Immune Globulin.

For instance, an insoluble cross-linked agarose gel such as Sepharose (Pharmacia Fine Chemicals Co.), is reacted with cyanogen bromide, and then with purified inactivated hepatitis B surface antigen (derived from plasma containing the antigen) which bonds covalently to the solid matrix. The matrix is then placed in a chromatography column, plasma or another physiological solution containing hepatitis B antibody is passed over the matrix in the column, and the matrix is then washed with a suitably buffered aqueous solution. At this point the hepatitis B antibody has become preferentially absorbed at the antigen sites on the column. The antibody is then eluted from the column, for instance with a solution of calcium ions, concentrated urea, or sodium thiocyanate, or other suitable elutant. The antibody is subsequently recovered from the elutant by dialysis, ultrafiltration, precipitation-redissolution, or a combination of these steps. The resultant product is characterized as purified monospecific hepatitis B antibody, containing a reducedamount of other antibodies encountered in the Hepatitis B Immune Globulin, and preferably containing essentially no non-hepatitic antibodies.

In accordance with this preferred embodiment, the biological product can be rendered non-infectious as to HBV by adding an effective amount of monospecific hepatitis B antibody in less than 1 mg, and even less than 0.5 mg, of material per ml of solution being treated. Less extraneous material, and in particular less non-hepatitic antibody, is thus administered to the patient.

The biological product to which the hepatitis B antibody has been added is then incubated, for at least half an hour and preferably an hour. The temperature is not critical, so long as it is high enough to permit the virus-antibody interaction to occur but not so high that unacceptable side reactions or deactivation of the antibody or the biological product occur. Room temperature incubation is preferred and highly satisfactory.

To render the biological product non-infectious as to HnAnBV, the incubated mixture is then dried by any means which removes the aqueous solvent without deactivating the hepatitis B antibody nor the underlying biological product. Lyophilization is the preferred drying method, in conventional laboratory lyophilizing equipment known in the art. This step permits the subsequent application of controlled heat to biological products such as Factor VIII and Factor IX which can readily be damaged by heat, especially when in aqueous solution. Lyophilization is typically carried out at a pressure of about 10 to 200 microns and a temperature of about -40°C to 40°C for about 24 to 96 hours. Residual moisture of up to about 0.1 to 0.2 wt. % can be tolerated.

The dried material is then heated under conditions effective to render the HnAnBV non-infectious, without substantially impairing the biological activity of the biological product. Preferably, the material is heated to a temperature of about 55° to about 70°C, and held at a temperature in this range for between about 10 hours and about 40 hours. More preferably, the material is held at about 60°C for about 30 hours. The desired temperatures can readily be maintained in a water bath equipped with a thermostatic control to maintain essentially constant temperature.

Heat treatment in the above preferred range is effective to retain at least about 10% of the biological activity of the product being treated. However, higher retention of activity can readily be attained, such as 25% and even over about 50%. Preferably, over 75% and even over 90% retention of activity, even with a normally heat-labile material such as Factor VIII, can readily be attained. In the most preferred embodiment, essentially no activity is lost. Another advantage of the process of this invention is that when the biological activity of the product is essentially completely retained, the biological product also undergoes essentially no denaturation. This is an important advantage because material that has been partially denatured can still possess biological activity, but can cause other side reactions in some patients to whom the material is administered. A further advantage is that the hepatitis B antibody remains active through the heat treatment for the HnAnBV.

The invention will be illustrated further in the following Examples.

## Example 1

This Example demonstrates that the heating process of the present invention does not adversely affect the ability of hepatitis B antibody to render HBV non-infectious, and that the heating regimen by itself is not effective against HBV in the absence of hepatities B antibody.

A batch of Factor VIII (aqueous solution) commercially manufactured in the United States was intentionally infected with a quantity of HBV (BOB-NIAD strain ay) such that when the product was placed in vials (30 ml per vial) each vial contained 3000 $CID_{50}$ of HBV. Some vials received 0.27 IU/ml (8.1 IU/vial) of hepatitis B antibody obtained by Cohn fractionation of plasma exhibiting high immune titer to hepatitis B surface antigen. All vials were incubated at room temperature for 1 hour, then lyophilized, and then heated in a water bath at 60 $\pm$ 0.5°C for 30 hours.

The activity of the antibody following the heating at 60°C for 30 hours, was assayed in representative vials and found to be 6.3 IU/Vial (0.21 IU/ml) which is acceptable retention of activity and within the limits of the assay.

A panel of 3 chimpanzees negative for signs of hepatitis were inoculated with the treated Factor VIII. One chimpanzee served as a positive control, and received treated Factor VIII to which the hepatitis B antibody had not been added. Two experimental chimpanzees received treated Factor VIII to which hepatitis B antibody had been added. The chimpanzees were observed for signs of hepatitis B infection, by determination of serum enzyme

0110407

levels (alanine aminotransferase, aspartate aminotransferase, gamma glutamyl transferase) on a weekly basis and liver biopsy samples were obtained for verification. The positive control readily contracted hepatitis in the normal incubation period of about 13 weeks. One of the experimental chimpanzees contracted hepatitis only after an additional period of 9 weeks following onset of the disease in the positive control; the length of this additional period is significant, and indicates that the antibody was effective notwithstanding the additional heating regimen. The other experimental chimpanzee failed to exhibit any signs of infection by hepatitis B, even 30 weeks following onset of the disease in the positive control.

Example II

This Example demonstrates that the heating process of the present invention is effective to render hepatitis non-A-non-B virus non-infectious.

. A batch of Factor VIII (aqueous solution) commercially manufactured in the United States was intentionally infected with a quantity of HnAnBV (NIAID Hutchinson pool, which is defined as containing $10^6$ Chimpanzee Hepatitis Doses (CHD) per ml in the undiluted state) such that when the product was placed in vials (30 ml each) each vial contained $10^{3.5}$ CHD of HnAnBV. The vials were lyophilized. Some vials were subjected to heating at 60 ± 0.5°C for 30 hours in a water bath. Replicate vials were retained as positive controls.

A panel of 5 chimpanzees negative for signs for hepatitis were inoculated with samples of the nAnB-infected Factor VIII. Two chimpanzees were positive controls and received unheated vials; three chimpanzees were experimental and received heated vials. The chimpanzees were observed for signs of hepatitis nAnB infection, by determination of serum enzyme levels (alanine aminotransferase, aspartate aminotransferase, gamma glutamyl transferase) on a weekly basis, and liver biopsy samples were obtained for verification. Chimpanzees which received heated vials of Factor VIII did not develop signs of hepatitis disease in the expected incubation time interval of 7 to 9 weeks, nor thereafter. Two of the chimpanzees which had received heated vials were rechallenged in the twenty-third week following inoculation with the heated Factor VIII, and both then developed hepatitis disease within a 7 to 9 week incubation period. One control chimpanzee developed hepatitis disease following a normal incubation period after receipt of unheated HnAnBV-infected Factor VIII. The other control did not develop the disease, and on subsequent rechallenge with the same virus used to rechallenge and infect the exper-mental chimpanzees, also did not develop the disease; this chimpanzee's behavior indicated therefore that failure to develop the disease was due to an anomaly as to the animal and not as to the virus or the heat treatment.

### Example III

Batches of the same commercial Factor VIII as used in Examples I and II were heated to 60 ± 0.5°C for 30 hours without addition of hepatitis virus or antibody. Following heating, the Factor VIII was then tested in comparison to unheated commercial Factor VIII, and was found to have undergone no change in chemical and biological properties detectable to the limits of the analytical techniques employed, when tested as to reconstitution characteristics, biological activity, stability, gel electrophoresis, immunochemical identity, light absorbence, _in vivo_ tolerance, _in vivo_ recovery, and _in vivo_ half-life.

WHAT IS CLAIMED IS:

1. A process for preventing the transmission of hepatitis B virus and hepatitis non-A-non-B virus by an injectable biological product containing said viruses, comprising

(a) incubating the biological product *in vitro* in solution with an amount of hepatitis B antibody effective to render the biological product non-infectious with respect to hepatitis B,

(b) drying the mixture formed in step (a) under conditions which are non-destructive to the biological product, and

(c) heating the dried mixture formed in step (b) under conditions effective to render the biological product non-infectious with respect to hepatitis non-A-non-B, while retaining at least about 10% of the biological activity of the biological product.

2. The process of claim 1 wherein in step (b) the mixture is dried by lyophilization.

3. The process of claim 1 wherein the biological product undergoes essentially no denaturation.

4. The process of claim 1 wherein the biological product is derived from pooled plasma.

5. The process of claim 4 wherein the biological product is blood fractions, blood sera containing plasma protein components, or placenta plasma proteins.

6. The process as in any of claims 1-5 wherein the biological product retains at least about 90% of its biological activity.

7. The process as in any of claims 1-5 wherein the biological product retains essentially all its biological activity.

0110407

8. The process as in any of claims 1-7 wherein the amount of hepatitis B antibody in step (a) comprises about 0.1 to about 20 IU per ml of solution.

9. The process of claim 8 wherein the hepatitis B antibody is added in a composition thereof comprising a total of no more than about 1 mg of antibody-bearing material per ml of solution.

10. The process as in any of claims 1-9 wherein in step (c) the mixture is held at a temperature of about 55°C to about 70°C for about 10 hours to about 40 hours.

11. The process as in any of claims 1-9 wherein in step (c) the mixture is held at about 60°C for about 30 hours.

12. Injectable biological products produced by the process of any of claims 1-11.